# EUROPEAN PATENT SPECIFICATION

(11) **EP 3 372 221 B1**
(45) Date of publication and mention of the grant of the patent: **20.11.2019**
(21) Application number: 18158525.8
(22) Date of filing: 26.02.2018
(51) Int. Cl.: A61K 8/37, A61K 8/49, A61K 8/67, A61Q 19/00, A61K 8/92, A61K 8/04

(54) **HYDROPHOBIC GEL BASED ON VITAMIN E FREE FROM SILICONE PRODUCTS FOR TOPICAL APPLICATION**
SILIKONPRODUKTFREIES HYDROPHOBES GEL AUF BASIS VON VITAMIN E ZUR TOPISCHEN ANWENDUNG
GEL HYDROPHOBE À BASE DE VITAMINE E EXEMPT DE PRODUITS À BASE DE SILICONE POUR APPLICATION TOPIQUE

(30) Priority: 06.03.2017 IT 201700024605
(43) Date of publication of application: 12.09.2018
(73) Proprietor: BIO.LO.GA. S.r.l., 31015 Conegliano (TV) (IT)
(72) Inventor: PANIN, Giorgio, 45100 Rovigo (IT)
(74) Representative: Ferreccio, Rinaldo

(56) References cited:
- EP-A1- 0 998 943
- WO-A1-98/10793
- WO-A1-2007/003658

## Description

### FIELD OF THE INVENTION

The present invention generally relates to a formulation for topical use and in particular to a preparation in the form of a hydrophobic gel (lipogel) based on vitamin E, free of silicone products.

### PRIOR ART

Vitamin E and derivatives thereof are substances that are widely used in the pharmaceutical and cosmetic industry, by virtue of their antioxidant properties and their scavenging properties towards free radicals, in the preparation of formulations for the treatment of skin diseases, or to combat or prevent skin blemishes.

Vitamin E, particularly in its form of tocopheryl acetate, is easy to spread, is absorbed with surprising rapidity, does not give rise to unpleasant sensations of heat, leaves the skin soft, elastic and not sticky, and is resistant to cleansing with water or detergents. Moreover, owing to the fact that vitamin E acetate is not a molecule foreign to the human organism, it can easily be integrated in the lipids present in the stratum corneum and can facilitate absorption of substances dispersed therein through the skin.

Cosmetic skin care compositions associating vitamin E with moisturizing substances are widely known: stratum corneum hydration is key for healthy skin, largely determining barrier and permeation/perspiration properties thereof. These compositions have predominantly an aqueous or a hydrolipidic base: e.g. emulsions, aqueous gels, foams, etc., wherein the moisturizing action is exerted by the aqueous component.

There are also topical compositions with a hydrophobic base, i.e. water-free compositions, mainly used for lips care or as balms. In the absence of water in the formulation, moisturization is indirectly obtained herein, by making the treated area more elastic and more permeable to environmental moisture.

For example, patent application FR 2978661 describes a balm almost exclusively consisting of neutral and polar lipids, with small amounts (0.1-2%) of active ingredients, e.g. Vitamin E. Patent application CN 102688162 discloses a gloss formulation including shea butter, triglyceride of caprylic and capric acid and active ingredient in small amounts, e.g. 0.5% of vitamin E. Patent application US 2013/319889 discloses compositions for skin care containing beeswax, cocoa butter, shea butter, a complex mixture of vegetable oils, together with very small amounts of active ingredient, e.g. Vitamin E.

In all the aforementioned publications, the authors did not study the incorporation of high amounts of active ingredient into the composition, nor the resulting compositions stability; in the majority of the cases these are compositions with a highly variable viscosity depending on temperature, with an oily texture when in contact with the skin.

A modern and greatly appreciated form for skin moisturization by means of hydrophobic substances is that consisting of hydrophobic gel or lipo-gel: these are compositions having low viscosity, marginally affected by ambient temperature, easily applicable also onto large skin surfaces, and capable of forming thereon a thin and stable layer that protects skin and maintains the composition on the application site, extending in time the elasticizing and moisturizing effect. Unlike fat-based creams, the lipogel does not have an oily texture when it comes in contact with the skin, therefore giving the user a more pleasant feeling upon application thereof; in particular the lipogel does not tend to fluidize at skin temperature: in fact, its texture is not regulated by the viscosity of the fats therein contained, but by the formation of a three-dimensional molecular lattice (gel) which reduces the density of the product and ensures its structuring in a substantially temperature-independent manner.

For example, application WO 98/10793 to the same applicant describes a hydrophobic gel for topical use, consisting of Vitamin E and a cyclomethicone:dimethiconol mixture in 8:2 ratio by weight, with or without hydrogenated castor oil; in particular two examples of formulation, wherein high quantities of vitamin E (20 or 30% of the composition total weight) have been used in association with a 8:2 cyclomethicone/dimethiconol mixture and optionally 5% hydrogenated castor oil, were provided.

Such gels proved to be excellent formulations for cosmetic use; however, over time, they showed a certain tendency to separate a liquid phase (syneresis).

This issue has been overcome by the hydrophobic gels subsequently described in patent application EP 998 943, on behalf of the Applicant, where stable hydrophobic gels, based on vitamin E acetate, a volatile silicone, and hydrogenated castor oil are described; a limitation for these compositions is represented by the use of significant percentages of synthetic silicone products (cyclomethicone, hexamethyldisiloxane, etc.), which can represent up to 70% by weight of the composition: these are non-natural elements for the skin, possibly related to sensitization events and known cause of environmental toxicity. On the other hand, it is not easy to remove these components, which enable adequate skin moisturization and form stable gels with high amounts of vitamin E. Therefore, there still is the need for new hydrophobic gel formulations, incorporating high amounts of vitamin E, which avoid the use of silicone products and use natural components, same or similar to those naturally occurring in the skin, and which show a high moisturizing activity.

### SUMMARY

In view of these needs, the Applicant has now developed a new formulation for topical use in which specific hydrophobic components, in specific concentration ranges, cooperate in a synergistic manner to provide a hydrophobic gel (lipogel) with high amounts of vitamin E, high moisturizing power, without the use of silicone products, and highly akin to skin composition.

The new formulation in question, entirely based on natural substances, comprises, in percentages by weight on the total formulation weight:
- from 3 to 65% of vitamin E,
- from 20 to 60% of a vegetable butter or a wax
- from 10 to 30% of a triglyceride of caprylic and capric acid
- from 3 to 20% of a gelling agent for lipids selected from triglyceride of palmitic and stearic acid and sorbitan olivate.

Preferably, the formulation according to the invention comprises, in percentages by weight on the total formulation weight,
- from 20 to 40% of vitamin E,
- from 20 to 30% of a vegetable butter or a wax,
- from 15 to 25% of a triglyceride of caprylic and capric acid,
- from 3 to 16% of said gelling agent for lipids.

Preferably, the gelling agent for lipids is sorbitan olivate.

In an aspect thereof, the present formulation further comprises one or more ingredients selected among hydrogenated castor oil, phytosterols, and ceramide.

The hydrogenated castor, if any, is contained in a percentage from 1 to 10%; ceramide, if any, is contained in a percentage from 0.01 to 1% and the phytosterols, if any, are contained in a percentage from 0.1 to 12%, on the total weight of the formulation.

Preferably, the vitamin E is tocopheryl acetate, the vegetable butter is shea butter, and the ceramide is ceramide-NP.

A particularly preferred composition according to the present invention comprises:
- 25-35% of tocopheryl acetate,
- 20-30% of shea butter,
- 18-25% of triglyceride of caprylic and capric acid,
- 5-16% of sorbitan olivate,
- 0.1-0.4% of ceramide-NP,
- 2-10% of phytosterols.

In an aspect thereof, the present invention concerns a process for preparing a hydrophobic gel as disclosed above, comprising mixing said vitamin E, vegetable butter or wax, triglyceride of caprylic and capric acid, gelling agent, and possible ceramide, phytosterols and hydrogenated castor oil, in the aforementioned percentages.

In a further aspect thereof, the present invention concerns the use of a formulation as disclosed above, as a cosmetic topical skin moisturizing and protective product.

In experimental tests carried out by the Applicant, the formulation according to the present invention proved to be extremely effective in promoting skin moisturization, with a marked effect after few hours from administration and lasting many hours without the need for new application, even more effective than currently known hydrophobic gels.

### DETAILED DESCRIPTION

The term "free" of silicone products, as used herein with reference to present formulations, means that said silicone products are absent from the formulations or may be present only in trace amounts or as impurities, not affecting the formulation properties. In turn, the term "silicone products" refers to any polymeric product containing silicon; the term is particularly but not exclusively related to products commonly found in topical compositions, such as silicone, dimethicone, cyclomethicone, dimethiconol, polysiloxanes, etc.

The term "hydrophobic" with reference to the present formulations means that they do not contain water or other aqueous or polar solvent in any state, e.g. free or emulsified, including instead the hydrophobic substances listed in the present invention.

In the present compositions, the content of vitamin E is comprised between 3 and 65%, preferably comprised between 20 and 40%, for example between 25-35% by weight on the composition weight. Vitamin E can be used in all its forms (tocopherols and tocotrienols, isomers (alpha, beta, gamma, delta) and derivatives thereof. The use of vitamin E in the form of tocopheryl acetate is preferred. Vitamin E gives to the present compositions useful skin protective properties, reducing radical oxidation events, preventing aging phenomena, and contributing to skin covering layer softness by means of the product left on the skin after administration.

The vegetable butter used in these formulations can be any of those commonly available, such as butter of: shea, cocoa, almonds, kokum, green tea, apricot, orange, lemon, pistachio, coffee, etc. Shea butter is particularly preferred. Wax also can be chosen from among those commonly available; preferred waxes are rice wax and beeswax. Vegetable butter or wax is employed in an amount from 20 to 60%, preferably from 20 to 40%, advantageously 20-30%.

The triglyceride of caprylic and capric acid (INCI Caprylic/Capric Triglyceride) is a synthetic glycerol triester with C8-C10 acids - caprylic (C8) and capric acid (C10) derived from coconut oil fractionation. It is a colorless to slightly yellow, low viscosity, odourless liquid. It is a good substitute for vegetable oils and is stable to oxidation with respect to the latter because it is completely saturated. It has remarkable emollient properties. This product is employed in the present formulations in an amount comprised between 10 to 30%, preferably between 15 to 25%.

The gelling agent for lipid is selected among a palmitic and stearic acid triglyceride (INCI Palmitic/Stearic Triglyceride) and sorbitan olivate (INCI Sorbitan Olivate).

The palmitic and stearic acid triglyceride has also emollient and antioxidant skin properties; it is a highly environmentally friendly product, stable to oxidation as it is completely saturated. The product is commercially available from various sources, e.g. under the brand Olifeel® (pearls). The preferred gelling agent is sorbitan olivate, which gives the lipogel a pleasant silky touch, moisturizing properties and good spreadability. The product is commercially available from various sources, e.g. with the Olivem 900® trade mark.

The gelling agent is used in the formulations in an amount comprised between 3 and 20%, preferably between 3 and 16%.

The present formulations may optionally contain ceramide and/or phytosterols. Ceramide is a waxy lipid consisting of sphingosine and fatty acids; it is typically present in the stratum corneum, where it prevents dehydration events and increases the barrier function. Nine natural ceramides are known, all of which can be used according to the invention, either alone or mixed together. Particularly preferred is ceramide-NP or ceramide-3, consisting of N-acyl sphingosine and non-hydroxylated fatty acids.

Phytosterols are a group of plant steroids, with structure similar to cholesterol. Stigmasterol, sitosterol, campesterol, etc., which can be used alone or combined, are typical members of this class. Preferred compositions may comprise from 0.01 to 1% of ceramide (preferably from 0.1 to 0.4%) by weight on total composition. They may further comprise from 0.1 to 12%, preferably 2-10%, of phytosterols by weight on total composition.

Optionally, the formulations of the invention may include additional ingredients, either as excipients or further active ingredients. Among the excipients in particular hydrogenated castor oil is mentioned, preferably present as percentage from 1 to 10%, more preferably from 0.1 to 6% by weight of the composition. Additional excipients may be additional hydrophobic components, rheology modifiers, preservatives, perfumes, etc. Further hydrophobic components are, for example, vegetable oils and fatty acids esters such as octyl palmitate, isopropyl myristate and ethyl oleate or mixtures thereof.

The hydrophobic gel according to the invention effectively dissolve or suspend pharmaceutically active principles, also in large quantities.

Examples of active ingredients that can be used (in addition to vitamin E) are: antibiotics, such as gentamicin, neomycin, clindamycin and tetracyclines, corticosteroids, such as hydrocortisone acetate or butyrate, diflucortolone valerate, methylprednisolone aceponate, mometasone furoate and betamethasone esters, trans-retinoic acid, synthetic retinoids, calcipotriol, vitamins such as retinol and its derivatives (retinol acetate and palmitate), ascorbic acid lipophilic derivatives, such as palmitoyl ascorbic acid, vitamin K, vitamin D, vaso-protectors, such as flavonoids and topical anti-inflammatory agents. Lipophilic active substances are preferably used, which effectively dissolve in the current hydrophobic medium; however, the addition of hydrophilic active substances, in this case suspended or otherwise incorporated within the composition, is not excluded.

The invention includes a process for preparing the hydrophobic gel described above; in a most general sense, the process comprises mixing together said vitamin E, vegetable butter or wax, gelling agent and any ceramides, phytosterols and hydrogenated castor oil, in aforementioned percentages thereof.

The invention also covers the use of the previously described hydrophobic lipogel as topical skin moisturizing and protective cosmetic product.

The hydrophobic gel according to the invention has excellent stability, excellent spreadability on skin, and is rapidly absorbed. Following the application of the hydrophobic gel according to the invention, the skin is extremely soft and silky. Compared to the known lipogels, it allows to earlier achieve a substantial moisturizing effect, which is maintained for many hours after application.

For further illustration of the present invention, some non-limiting preparation examples and efficacy proofs of the hydrophobic gel according to the invention are provide hereinafter.

### EXAMPLE 1 Lipogel preparation

A formulation in accordance with the invention was prepared according to the following composition, where the percentages are intended by weight of the total composition.
- tocopheryl acetate: 30%
- shea butter: 24%
- triglyceride of caprylic and capric acid: 21.7%
- sorbitan olivate: 14%
- phytosterols: 10%
- ceramide-NP: 0.3%

The preparation method is as follows: preparing a first phase comprising Vitamin E, phytosterols and ceramides by heating the whole up to 120°C to obtain a homogeneous solution. In a separate container the missing ingredients are heated up to 60°C. The two phases are then combined together and mixed for about 30 minutes at room temperature.

### EXAMPLE 2: Evaluation of moisturizing effect

### 2.1 Corneometer

The corneometer is a device for measuring the moisture content of the surface layers of epidermis.

It consists of a probe which, resting on the skin area of interest, measures skin surface electrical conductance, which varies depending on skin moisture content.

This means that the device exploits the physical principle whereby the skin surface area (stratum corneum) displays an electrical resistance to the current flow, which is the lower, the more the skin is hydrated. The method is not affected by other components within in the stratum corneum (e.g. salts). The assay is carried out by placing on the skin surface a probe, which is run through by a very weak electric current, and subsequently reading the corresponding conductance value, which is related to the water content of the epidermis at the measuring point.

### 2.2 Test Objectives

The purpose of the test is the assessment of the moisturizing capability of the composition of example 1 (Vea LIPO 3 Formula 2) over 8 hours period, compared to untreated subjects (negative control) or to subjects treated with an anhydrous lipogel based on silicone (Vea Lipogel), as reference product described in patent application EP 998 943, par. [0023].

### 2.3 Materials and Methods

A prototype batch of VEA LIPO3 Formula2 has been used for the test. The evaluation was performed with Corneometer CM825. The test was performed on 10 volunteers, who have signed and dated Informed Consent and Privacy Policy modules before the assay.

Each volunteer was asked to sit in the laboratory for at least 15 minutes. The investigator performed a measurement of the baseline moisturization level in three areas of both right and left volar forearm, before applying the investigational product. Each area has a size of 5x2 cm. 5 measurements were collected from each area. The mean of the measurements for each area at each time (T0, T4 and T8) has been considered.

After this measurement, the investigator applied:
- in an area A: VEA LIPO3 Formula 2
   in an area B, different from the previous one: VEA LIPOGEL
- a third area C, untreated throughout the duration of the assay, served as a negative control.

Two checks have then been carried out at 4 hours distance. At each check, the volunteer was asked to wait in the room for at least 15 minutes before performing the measurements required in all three areas.

### 3. Results

An acronym legend used to identify the areas, is depicted below:
A = VEA LIPO3 Formula 2 application area
B = VEA LIPOGEL application area
C = area used as a negative control
T0 = mean of 5 measurements carried out before product application
T4 = mean of 5 measurements taken 4 hours after first application
T8 = mean of 5 measurements taken 8 hours after first application. The values obtained from the measurement carried out at 4 and 8 hours from test start were compared with the baseline measurements (TO).

| **AREA A (VEA LIPO3 FORMULA2)** | | | |
|---|---|---|---|
| **Volunteer** | **T0** | **T4** | **T8** |
| 1 | 22.40 | 29.70 | 23.94 |
| 2 | 22.42 | 29.88 | 30.93 |
| 3 | 27.65 | 29.15 | 27.17 |
| 4 | 21.60 | 24.12 | 26.53 |
| 5 | 25.03 | 29.50 | 28.64 |
| 6 | 27.53 | 34.63 | 33.15 |
| 7 | 26.35 | 35.98 | 33.90 |
| 8 | 28.90 | 41.52 | 27.34 |
| 9 | 43.10 | 55.00 | 57.92 |
| 10 | 18.83 | 27.65 | 30.50 |
| **Mean** | **26.38** | **33.71** | **32.00** |

| **AREA B (VEA LIPOGEL)** | | | |
|---|---|---|---|
| **Volunteer** | **T0** | **T4** | **T8** |
| 1 | 32.23 | 32.08 | 30.00 |
| 2 | 25.50 | 29.43 | 32.05 |
| 3 | 27.70 | 28.49 | 28.07 |
| 4 | 17.98 | 19.43 | 17.00 |
| 5 | 23.53 | 26.84 | 28.28 |
| 6 | 31.55 | 37.70 | 35.65 |
| 7 | 21.28 | 25.68 | 26.28 |
| 8 | 21.82 | 24.93 | 29.10 |
| 9 | 39.03 | 46.67 | 52.77 |
| 10 | 30.38 | 35.57 | 34.78 |
| **Mean** | **27.10** | **30.68** | **31.40** |

| **AREA C (NEGATIVE CONTROL)** | | | |
|---|---|---|---|
| **Volunteer** | **T0** | **T4** | **T8** |
| 1 | 21.60 | 20.86 | 22.20 |
| 2 | 25.72 | 27.90 | 27.65 |
| 3 | 25.72 | 26.24 | 26.48 |
| 4 | 30.73 | 30.76 | 30.80 |
| 5 | 29.33 | 32.90 | 32.38 |
| 6 | 30.90 | 32.73 | 33.97 |
| 7 | 23.24 | 25.03 | 23.48 |
| 8 | 27.10 | 28.53 | 27.40 |
| 9 | 39.02 | 45.50 | 45.18 |
| 10 | 30.95 | 32.14 | 32.48 |
| **Mean** | **28.43** | **30.26** | **30.20** |

The data obtained from VEA LIPO3 Formula 2 and VEA LIPOGEL application area then were compared with data detected within C control area, respectively, obtaining the following results.

| **Comparison of VEA LIPO3 Formula 2 and Negative Control at T4** | | | |
|---|---|---|---|
| | **AREA A** | **AREA C** | **AREA A/AREA C** |
| **Volunteer** | **T4/T0** | **T4/T0** | |
| 1 | 1.33 | 0.97 | 1.37 |
| 2 | 1.33 | 1.08 | 1.23 |
| 3 | 1.05 | 1.02 | 1.03 |
| 4 | 1.12 | 1.00 | 1.12 |
| 5 | 1.18 | 1.12 | 1.05 |
| 6 | 1.26 | 1.06 | 1.19 |
| 7 | 1.37 | 1.08 | 1.27 |
| 8 | 1.44 | 1.05 | 1.36 |
| 9 | 1.28 | 1.17 | 1.09 |
| 10 | 1.47 | 1.04 | 1.41 |
| **Mean** | | | **1.21** |

| **Comparison of VEA LIPOGEL and Negative Control at T4** | | | |
|---|---|---|---|
| | **AREA B** | **AREA C** | **AREA B/AREA C** |
| **Volunteer** | **T4/T0** | **T4/T0** | |
| 1 | 1.00 | 0.97 | 1.03 |
| 2 | 1.15 | 1.08 | 1.06 |
| 3 | 1.03 | 1.02 | 1.01 |
| 4 | 1.08 | 1.00 | 1.08 |
| 5 | 1.14 | 1.12 | 1.02 |
| 6 | 1.19 | 1.06 | 1.13 |
| 7 | 1.21 | 1.08 | 1.12 |
| 8 | 1.14 | 1.05 | 1.09 |
| 9 | 1.20 | 1.17 | 1.03 |
| 10 | 1.17 | 1.04 | 1.13 |
| **Mean** | | | **1.07** |

| **Comparison of VEA LIPO3 Formula 2 and Negative Control at T8** | | | |
|---|---|---|---|
| | **AREA A** | **AREA C** | **AREA A/AREA C** |
| **Volunteer** | **T8/T0** | **T8/T0** | |
| 1 | 1.07 | 1.03 | 1.04 |
| 2 | 1.38 | 1.08 | 1.28 |
| 3 | 0.98 | 1.03 | 0.95 |
| 4 | 1.23 | 1.00 | 1.23 |
| 5 | 1.14 | 1.10 | 1.04 |
| 6 | 1.20 | 1.10 | 1.10 |
| 7 | 1.29 | 1.01 | 1.27 |
| 8 | 0.95 | 1.01 | 0.94 |
| 9 | 1.34 | 1.16 | 1.16 |
| 10 | 1.62 | 1.05 | 1.54 |
| **Mean** | | | **1.15** |

| **Comparison of VEA LIPOGEL and Negative Control at T8** | | | |
|---|---|---|---|
| | **AREA B** | **AREA C** | **AREA B/AREA C** |
| **Volunteer** | **T8/T0** | **T8/T0** | |
| 1 | 0.93 | 1.03 | 0.91 |
| 2 | 1.26 | 1.08 | 1.17 |
| 3 | 1.01 | 1.03 | 0.98 |
| 4 | 0.95 | 1.00 | 0.94 |
| 5 | 1.20 | 1.10 | 1.09 |
| 6 | 1.13 | 1.10 | 1.03 |
| 7 | 1.23 | 1.01 | 1.22 |
| 8 | 1.33 | 1.01 | 1.32 |
| 9 | 1.35 | 1.16 | 1.17 |
| 10 | 1.14 | 1.05 | 1.09 |
| **Mean** | | | **1.09** |

In summary, the results obtained show that VEA LIPO3 Formula2 after 4 hours has a greater moisturizing effect than VEA LIPOGEL (mean of 1.21 compared to 1.07) with an overall increase of 13.56%.

The greater moisturizing effect of VEA LIPO 3 Formula 2 with respect to VEA LIPOGEL is present even 8 hours afterapplication, at which time an overall increase of 5.76 has been verified.. From these data it can reasonably be deduced that VEA LIPO3 Formula 2 is absorbed faster than VEA LIPOGEL, thus exerting faster and more intensively its moisturizing effect, which is maintained higher than that of VEA LIPOGEL even 8 hours after application.

## Claims

1. A hydrophobic gel formulation for topical use, free of silicone products, comprising in weight percentage on the total weight of the formulation:
- from 3 to 65% of vitamin E,
- from 20 to 60% of a vegetable butter or a wax,
- from 10 to 30% of a triglyceride of caprylic and capric acid,
- from 3 to 20% of a gelling agent for lipids selected from triglyceride of palmitic and stearic acid and sorbitan olivate.

2. The formulation according to claim 1, comprising in weight percentage on the total weight of the formulation:
- from 20 to 40% of vitamin E,
- from 20 to 30% of a vegetable butter or a wax,
- from 15 to 25% of a triglyceride of caprylic and capric acid,
- from 3 to 16% of said gelling agent for lipids.

3. The formulation according to claims 1-2, wherein the gelling agent for lipids is sorbitan olivate.

4. The formulation according to claims 1-3, further comprising one or more among hydrogenated castor oil, phytosterols, and ceramide.

5. The formulation according to claim 4, wherein the hydrogenated castor oil is present in a percentage from 1 to 10% by weight, ceramide from 0.01 to 1% by weight and the phytosterols from 0.1 to 12% by weight, on the total weight of the formulation.

6. The formulation according to claims 1-5, wherein said vitamin E is tocopheryl acetate, said vegetable butter is shea butter, and said ceramide is ceramide-NP.

7. The formulation according to claims 1-6, comprising in weight percentage on the total weight of the formulation:
- 25-35% of tocopheryl acetate,
- 20-30% of shea butter,
- 18-25% of triglyceride of caprylic and capric acid,
- 5-16% of sorbitan olivate,
- 0.1-0.4% of ceramide-NP,
- 2-10% of phytosterols.

8. A process for preparing a hydrophobic gel according to claims 1-7, comprising mixing said vitamin E, vegetable butter or wax, triglyceride of caprylic and capric acid, gelling agent, and possible ceramide, phytosterols and hydrogenated castor oil, in said percentages thereof.

9. Use of a formulation according to claims 1-7, as a cosmetic topical skin moisturizing product.

## Patentansprüche

1. Eine silikonproduktfreie hydrophobe Gelformulierung zur topischen Anwendung, umfassend in Gewichtsprozent auf das Gesamtgewicht der Formulierung bezogen:
- 3 bis 65% Vitamin E,
- 20 bis 60% einer pflanzlichen Butter oder eines Wachses,
- 10 bis 30% eines Triglycerids von Capryl- und Caprinsäure,
- 3 bis 20% eines Geliermittels für Lipide, ausgewählt aus der Gruppe Triglycerid der Palmitin- und Stearinsäure und Sorbitanolivat.

2. Die Formulierung nach Anspruch 1, umfassend in Gewichtsprozent auf das Gesamtgewicht der Formulierung bezogen:
- 20 bis 40% Vitamin E,
- 20 bis 30% einer pflanzlichen Butter oder eines Wachses,
- 15 bis 25% eines Triglycerids von Capryl- und Caprinsäure,
- 3 bis 16% des Geliermittels für Lipide.

3. Die Formulierung nach den Ansprüchen 1-2, wobei das Geliermittel für Lipide Sorbitanolivat ist.

4. Die Formulierung nach den Ansprüchen 1-3, weiter umfassend eines oder mehrere aus der Gruppe von hydriertem Rizinusöl, Phytosterin, und Ceramide.

5. Die Formulierung nach Anspruch 4, wobei, auf das Gesamtgewicht der Formulierung bezogen, vorhanden ist mit einem Anteil von 1 bis 10 Gew.% hydriertes Rizinusöl, 0.01 bis 1 Gew.% Ceramide und 0.01 bis 12 Gew.% Phytosterin.

6. Die Formulierung nach den Ansprüchen 1-5, wobei das Vitamin E Tocopherylacetat ist, die pflanzliche Butter Sheabutter ist, und das Ceramide Ceramide-NP ist.

7. Die Formulierung nach den Ansprüchen 1-6, umfassend in Gewichtsprozent auf das Gesamtgewicht der Formulierung bezogen:
- 25-35% Tocopherylacetat,
- 20-30% Sheabutter,
- 18-25% Triglyceridsvon Capryl- und Caprinsäure,
- 5-16% Sorbitanolivat,
- 0.1-0.4% Ceramide-NP,
- 2-10% Phytosterin.

8. Verfahren für die Vorbereitung eines hydrophoben Gels nach den Ansprüchen 1-7, umfassend, ein Mischen von Vitamin E, pflanzlicher Butter oder Wachs, Triglycerid von Capryl- und Caprinsäure, Geliermittel und möglichem Ceramide, Phytosterin und hydriertem Rizinusöl in den genannten Prozentsätzen davon.

9. Verwendung einer Formulierung nach den Ansprüchen 1-7 als ein kosmetisches topisches Hautbefeuchtungsprodukt.

## Revendications

1. Formulation de gel hydrophobe pour une utilisation topique, dépourvue de produits de silicone, comprenant en pourcentage en poids sur le poids total de la formulation :
- de 3 à 65 % de vitamine E,
- de 20 à 60 % d'un beurre végétal ou d'une cire,
- de 10 à 30 % d'un triglycéride d'acide caprylique et caprique,
- de 3 à 20 % d'un agent gélifiant pour les lipides sélectionnés parmi un triglycéride d'acide palmitique et stéarique et l'olivate de sorbitane.

2. Formulation selon la revendication 1, comprenant en pourcentage en poids sur le poids total de la formulation :
- de 20 à 40 % de vitamine E,
- de 20 à 30 % d'un beurre végétal ou d'une cire,
- de 15 à 25 % d'un triglycéride d'acide caprylique et caprique,
- de 3 à 16 % dudit agent gélifiant pour les lipides.

3. Formulation selon les revendications 1 et 2, dans laquelle l'agent gélifiant pour les lipides est l'olivate de sorbitane.

4. Formulation selon les revendications 1 à 3, comprenant en outre un ou plusieurs parmi l'huile de ricin hydrogénée, les phytostérols, et le céramide.

5. Formulation selon la revendication 4, dans laquelle l'huile de ricin hydrogénée est présente en un pourcentage de 1 à 10 % en poids, le céramide de 0,01 à 1 % en poids et les phytostérols de 0,1 à 12 % en poids, sur le poids total de la formulation.

6. Formulation selon les revendications 1 à 5, dans laquelle ladite vitamine E est l'acétate de tocophéryle, ledit beurre végétal est le beurre de karité, et ledit céramide est le céramide-NP.

7. Formulation selon les revendications 1 à 6, comprenant en pourcentage en poids sur le poids total de la formulation :
- 25 à 35 % d'acétate de tocophéryle,
- 20 à 30 % de beurre de karité,
- 18 à 25 % de triglycéride d'acide caprylique et caprique,
- de 5 à 16 % d'olivate de sorbitane,
- de 0,1 à 0,4 % de céramide-NP,
- de 2 à 10 % de phytostérols.

8. Procédé de préparation d'un gel hydrophobe selon les revendications 1 à 7, comprenant le mélange desdits vitamine E, beurre végétal ou cire, triglycéride d'acide caprylique et caprique, agent gélifiant, et éventuels céramide, phytostérols et huile de ricin hydrogénée, en pourcentages correspondants.

9. Utilisation d'une formulation selon les revendications 1 à 7, en tant que produit cosmétique topique hydratant pour la peau.
